(19)

Europäisches
Patentamt
European
Patent Office
Office européen
des brevets

(11) **EP 4 736 972 A1**

(12) **EUROPEAN PATENT APPLICATION**

(43) Date of publication:
**06.05.2026 Bulletin 2026/19**

(21) Application number: **25203242.0**

(22) Date of filing: **19.09.2025**

(51) International Patent Classification (IPC):
**A63F 13/798** (2014.01)   **A63F 13/214** (2014.01)
**A63F 13/42** (2014.01)   **A63F 13/44** (2014.01)
**A63F 13/46** (2014.01)

(52) Cooperative Patent Classification (CPC):
**A63F 13/214; A63F 13/42; A63F 13/44;
A63F 13/46; A63F 13/798**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH LA MA MD TN**

(30) Priority: **30.10.2024 IN 202421083425**

(71) Applicant: **Tata Consultancy Services Limited
Maharashtra (IN)**

(72) Inventors:
• **RADHAKRISHNAN, Anilraj
695581 Trivandrum, Kerala (IN)**
• **KUPPUSAMY SANTHANAM, Sivakumar
600086 Chennai, Tamil Nadu (IN)**
• **SIVAN, Vishnu
695581 Trivandrum, Kerala (IN)**
• **ANTONY, Grigary Cheeran
695581 Trivandrum, Kerala (IN)**
• **KURUP, Aravind Suresh
695581 Trivandrum, Kerala (IN)**

(74) Representative: **Goddar, Heinz J.
Boehmert & Boehmert
Anwaltspartnerschaft mbB
Pettenkoferstrasse 22
80336 München (DE)**

(54) **ELDER FRIENDLY DEVICE TO IMPROVE HAND EYE CO-ORDINATION USING AN ADAPTIVE GAMING TECHNIQUE**

(57) Existing therapeutic methods lack interactivity and adaptability to individual needs. The present disclosure retrieves from a database, a hardware component design as an interactive table comprising a grid of sensor(s) to detect input(s) from a user. The detected one or more inputs from user are passed to a gaming engine connected to the hardware component. Reflex time of user is recorded, when user provides the inputs to the sensors. Reflex time is compared with predefined time $T_n$ and at least one of following is performed based on comparison that includes incrementally changing position of object, resetting timer, increasing timer with a first delta value and moving position of object closer to initial position with a second delta value. An average response time, a progression metric and adjustments to timer are calculated. A cognitive function to assess the severity of cognitive decline of user is derived.

FIG. 4

EP 4 736 972 A1

**Description**

CROSS-REFERENCE TO RELATED APPLICATIONS AND PRIORITY

The present application claims priority to Indian application no. 202421083425, filed on October 30, 2024.

TECHNICAL FIELD

The disclosure herein generally relates to an interactive game system, and, more particularly, to an elder friendly device to improve hand eye co-ordination using an adaptive gaming technique.

BACKGROUND

A vast majority of the global population is ageing rapidly. Cognitive decline leads to many conditions including dementia which is a common condition among the elderly; Although the brains of patients with dementia gradually degrade, scientists have found that this degradation is still plastic. Therefore, in addition to slowing down the rate of degeneration with drugs, training can also keep the brain healthy. Further, as the body can be used to strengthen the physique through exercise, and often stimulate the brain to promote cognitive function, the brain can also be trained to improve cognitive function. Cognitive training can also be called brain gymnastics, which may help delay cognitive decline.

The conventional way to prevent cognitive decline is usually through similar rehabilitation equipment or a single training mode. The user's single training mode can be used for specific training for a certain cognitive function, if it needs to be diversified. Training various cognitive functions may require more time to perform various training modes and may require frequent visits to professional cognitive training in professional institutions, which can not only be inconvenient, but also impossible to effectively track training progress. Therefore, effective tracking of training progress and conducting preventive training in a convenient manner is one of the urgent problems in the field of cognitive training.

SUMMARY

Embodiments of the present disclosure present technological improvements as solutions to one or more of the above-mentioned technical problems recognized by the inventors in conventional systems. For example, in one embodiment, an elder friendly device to improve hand eye co-ordination using an adaptive gaming technique is provided. The method includes retrieving from a database, via one or more hardware processors, a hardware component design as an interactive table comprising a grid of one or more sensors arranged in a pre-defined matrix format; placing, via the one or more hardware processors, an object at an initial position specific to a position of the one or more sensors; detecting, via the one or more hardware processors, by the one or more sensors, one or more inputs obtained from a device associated with a user; sending, via the one or more hardware processors, by the one or more sensors, data pertaining to the detected one or more inputs obtained from the device associated with the user to a gaming engine connected to the designed hardware component; recording, via the one or more hardware processors, a reflex time pertaining to the user, when the user provides the one or more inputs to the one or more sensors; performing, via the one or more hardware processors, a comparison of the reflex time within a predefined time $T_n$, and performing one of: (i) incrementally changing the initial position of the object; (ii) resetting a timer $T_0$ to a predefined time interval; or (a) increasing the timer with a first delta value; and (b) moving the position of the object closer to the initial position with a second delta value; calculating, via the one or more hardware processors, at least one of the following based on the reflex time of the user: (i) an average response time over N inputs, wherein a response time $t_i$ of the user is recorded for each of the one or more inputs; (ii) a progression metric to evaluate an improvement based on a number of successful inputs from a number of total inputs; and (iii) one or more adjustments to the timer based on the progression metric; and deriving, via the one or more hardware processors, a cognitive function to assess a cognitive decline severity of the user based on the calculated average response time, the progression metric and the one or more adjustments.

In another aspect, there is provided a system to an elder friendly device to improve hand eye co-ordination using an adaptive gaming technique. The system comprises: a memory storing instructions; one or more communication interfaces; and one or more hardware processors coupled to the memory via the one or more communication interfaces, wherein the one or more hardware processors are configured by the instructions to: retrieve from a database, a hardware component design as an interactive table comprising a grid of one or more sensors arranged in a pre-defined matrix format. The system further includes placing an object at an initial position specific to a position of the one or more sensors; detecting by the one or more sensors, one or more inputs obtained from a device associated with a user; sending by the one or more sensors, data pertaining to the detected one or more inputs obtained from the device associated with the user to a gaming engine connected to the designed hardware component; recording a reflex time pertaining to the user, when the user provides the one or more inputs to the one or more sensors; performing a comparison of the reflex time within a predefined time $T_n$, and performing one of: (i) incrementally changing the initial position of the object; (ii) resetting a timer $T_0$ to a predefined time interval; or (a) increasing the timer with a first delta value; and (b) moving the position of the object closer to the initial position with a second delta value; calculating at least one of the following based on the reflex time of the user: (i) an average response time over N inputs, wherein a response time $t_i$ of the user is recorded for each of the one or more inputs; (ii) a progression metric to evaluate an improvement based on a number of successful inputs from a number of total inputs; and (iii) one or more adjustments to the timer based on the progression metric; and deriving a cognitive function to assess a cognitive decline severity of the user based on the calculated average response time, the progression metric and the one or more adjustments.

**In** yet another aspect, there are provided one or more non-transitory machine-readable information storage mediums comprising one or more instructions which when executed by one or more hardware processors cause retrieving from a database, a hardware component design as an interactive table comprising a grid of one or more sensors arranged in a pre-defined matrix format; placing an object at an initial position specific to a position of the one or more sensors; detecting by the one or more sensors, one or more inputs obtained from a device associated with a user; sending by the one or more sensors, data pertaining to the detected one or more inputs obtained from the device associated with the user to a gaming engine connected to the designed hardware component; recording a reflex time pertaining to the user, when the user provides the one or more inputs to the one or more sensors; performing a comparison of the reflex time within a predefined time $T_n$, and performing one of: (i) incrementally changing the initial position of the object; (ii) resetting a timer $T_0$ to a predefined time interval; or (a) increasing the timer with a first delta value; and (b) moving the position of the object closer to the initial position with a second delta value; calculating at least one of the following based on the reflex time of the user: (i) an average response time over N inputs, wherein a response time $t_i$ of the user is recorded for each of the one or more inputs; (ii) a progression metric to evaluate an improvement based on a number of successful inputs from a number of total inputs; and (iii) one or more adjustments to the timer based on the progression metric; and deriving a cognitive function to assess a cognitive decline severity of the user based on the calculated average response time, the progression metric and the one or more adjustments.

It is to be understood that both the foregoing general description and the following detailed description are exemplary and explanatory only and are not restrictive of the invention, as claimed.

## BRIEF DESCRIPTION OF THE DRAWINGS

The accompanying drawings, which are incorporated in and constitute a part of this disclosure, illustrate exemplary embodiments and, together with the description, serve to explain the disclosed principles:

FIG. 1 illustrates an exemplary system for an elder friendly device to improve hand eye co-ordination using an adaptive gaming technique, according to some embodiments of the present disclosure.
FIG. 2 is a functional block diagram of the system for the elder friendly device to improve hand eye co-ordination using an adaptive gaming technique, according to some embodiments of the present disclosure.
FIGS. 3A through 3C are flow diagrams illustrating the steps involved in the method to elder friendly device to improve hand eye co-ordination using an adaptive gaming technique, according to some embodiments of the present disclosure.
FIG. 4 shows a flow chart illustrating a hardware setup, a game flow, and a game set up in conjunction with the method to elder friendly device to improve hand eye co-ordination using an adaptive gaming technique, according to some embodiments of the present disclosure.

## DETAILED DESCRIPTION OF EMBODIMENTS

Exemplary embodiments are described with reference to the accompanying drawings. In the figures, the left-most digit(s) of a reference number identifies the figure in which the reference number first appears. Wherever convenient, the same reference numbers are used throughout the drawings to refer to the same or like parts. While examples and features of disclosed principles are described herein, modifications, adaptations, and other implementations are possible without departing from the scope of the disclosed embodiments.

Dementia can impair cognitive functions such as hand-eye coordination and reflexes. Existing therapeutic methods lack interactivity and adaptability to individual needs. The present disclosure addresses these issues by creating an adaptive game that not only provides engaging therapy but also monitors and assesses cognitive functions. The present disclosure retrieves a hardware component from a database, as an interactive table comprising a grid of one or more sensors arranged in a pre-defined matrix format. In the present method, the one or more sensors detect one or more inputs obtained from a device associated with a user and send the data pertaining to the detected one or more inputs obtained from the device associated with the user to a gaming engine connected to the designed hardware component. A reflex time of the user is recorded, when the user provides the one or more inputs to the one or more sensors. The reflex time is compared within a predefined time $T_n$ and at least one of the following steps are performed based on the comparison: (i) the position of the object is changed incrementally, (ii) a timer is reset to a predefined time interval, (iii) the timer is increased with a first delta value and (iv) the position of the object is moved closer to the initial position with a second delta value. Further the present disclosure calculates an average response time, a progression metric to evaluate improvement and one or more adjustments to the timer based on the reflex time of the user. Finally, a cognitive function to assess the health condition severity of the user is derived based on the calculated average response time, the progression metric and the one or more adjustments. The present disclosure provides adaptive feedback based on the user performance by adjusting the predefined time $T_n$.

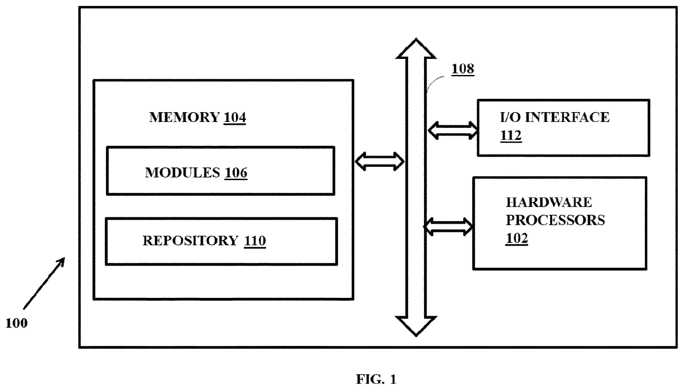

Referring now to the drawings, and more particularly to FIG. 1 through FIG. 4, where similar reference characters denote corresponding features consistently throughout the figures, there are shown preferred embodiments, and these embodiments are described in the context of the following exemplary system and/or method.

FIG. 1 illustrates an exemplary system for an elder friendly device to improve hand eye co-ordination using an adaptive gaming technique, according to some embodiments of the present disclosure. In an embodiment, the system 100 includes or is otherwise in communication with hardware processors 102, at least one memory such as a memory 104, and an I/O interface 112. The hardware processors 102, memory 104, and the Input /Output (I/O) interface 112 may be coupled by a system bus such as a system bus 108 or a similar mechanism. In an embodiment, the hardware processors 102 can be one or more hardware processors.

The I/O interface 112 may include a variety of software and hardware interfaces, for example, a web interface, a graphical user interface, and the like. The I/O interface 112 may include a variety of software and hardware interfaces, for example, interfaces for peripheral device(s), such as a keyboard, a mouse, an external memory, a printer and the like. Further, the I/O interface 112 may enable the system 100 to communicate with other devices, such as web servers, and external databases.

The I/O interface 112 can facilitate multiple communications within a wide variety of networks and protocol types, including wired networks, for example, local area network (LAN), cable, etc., and wireless networks, such as Wireless LAN (WLAN), cellular, or satellite. For the purpose, the I/O interface 112 may include one or more ports for connecting several computing systems with one another or to another server computer. The I/O interface 112 may include one or more ports for connecting several devices to one another or to another server.

The one or more hardware processors 102 may be implemented as one or more microprocessors, microcomputers, microcontrollers, digital signal processors, central processing units, node machines, logic circuitries, and/or any devices that manipulate signals based on operational instructions. Among other capabilities, the one or more hardware processors 102 is configured to fetch and execute computer-readable instructions stored in memory 104.

The memory 104 may include any computer-readable medium known in the art including, for example, volatile memory, such as static random-access memory (SRAM) and dynamic random-access memory (DRAM), and/or non-volatile memory, such as read only memory (ROM), erasable programmable ROM, flash memories, hard disks, optical disks, and magnetic tapes. In an embodiment, the memory 104 includes a plurality of modules 106. The memory 104 also includes a data repository (or repository) 110 for storing data processed, received, and generated by the plurality of modules 106.

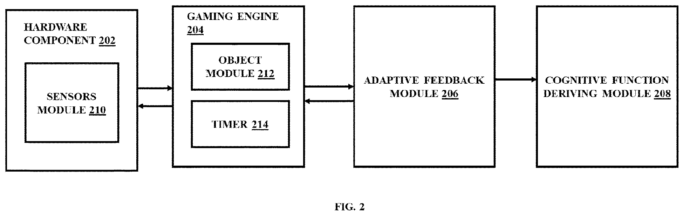

The plurality of modules 106 includes programs or coded instructions that supplement applications or functions performed by the system 100 for an elder friendly device to improve hand eye co-ordination using an adaptive gaming technique. The plurality of modules 106, amongst other things, can include routines, programs, objects, components, and data structures, which perform particular tasks or implement particular abstract data types. The plurality of modules 106 may also be used as signal processor(s), node machine(s), logic circuitries, and/or any other device or component that manipulates signals based on operational instructions. Further, the plurality of modules 106 can be used by hardware, by computer-readable instructions executed by the one or more hardware processors 102, or by a combination thereof. The plurality of modules 106 can include various sub-modules (not shown). The plurality of modules 106 may include computer-readable instructions that supplement applications or functions performed by the system 100 for an elder friendly device to improve hand eye co-ordination using an adaptive gaming technique. In an embodiment, the modules 106 include a hardware component 202, a gaming engine 204, an adaptive feedback module 206 and a cognitive function deriving module 208. The hardware component 202 comprises a sensors module 210. The gaming engine 204 comprises an object module 212 and a timer 214. The modules are depicted in FIG. 2. These modules that are depicted in FIG. 2 are implemented as at least one of a logically self-contained part of a software program, a self-contained hardware component, and/or a self-contained hardware component with a logically self-contained part of a software program embedded into each of the hardware component that when executed perform the above method described herein, in one embodiment of the present disclosure.

The data repository (or repository) 110 may include a plurality of abstracted pieces of code for refinement and data that is processed, received, or generated as a result of the execution of the module(s) 106.

Although the data repository 110 is shown internal to the system 100, it will be noted that, in alternate embodiments, the data repository 110 can also be implemented external to the system 100. The data contained within such an external database may be periodically updated. For example, new data may be added into the database (not shown in FIG. 1) and/or existing data may be modified and/or non-useful data may be deleted from the database. In one example, the data may be stored in an external system, such as a Lightweight Directory Access Protocol (LDAP) directory and a Relational Database Management System (RDBMS).

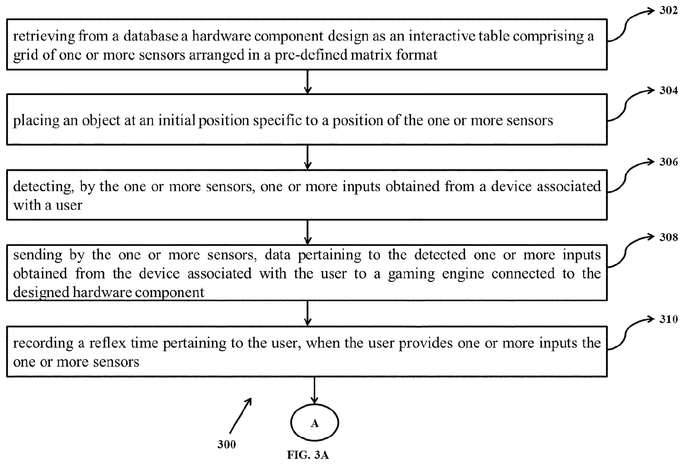

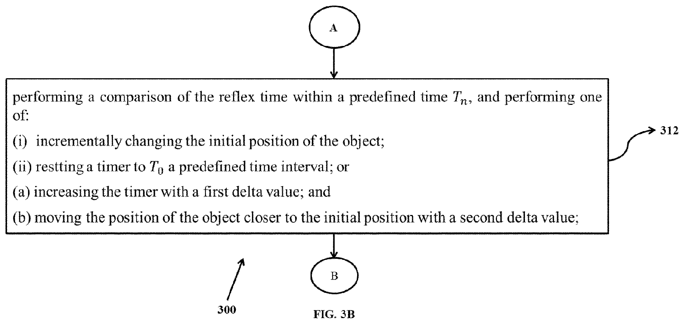

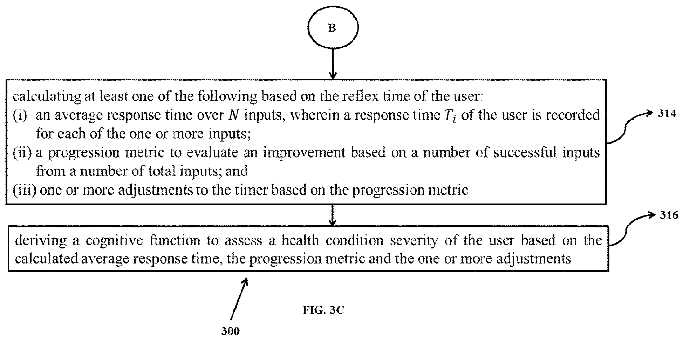

FIGS. 3A through 3C are flow diagrams illustrating a method for an elder friendly device to improve hand eye co-ordination using an adaptive gaming technique using the systems 100 of FIGS. 1-2, according to some embodiments of the present disclosure. Steps of the method of FIGS. 3A and 3C shall be described in conjunction with the components of FIG. 2. At step 302 of the method 300, the one or more hardware processors retrieves a hardware component (represented by the hardware component 202) from a database as an interactive table comprising a grid of one or more sensors (represented by the sensors module 210) arranged in a pre-defined matrix format.

The hardware component 202 is designed as an interactive table, where a 3x5 grid of sensors is arranged on top of a standard table. These one or more sensors detect when the user taps on them, and the readings are sent to the software component (represented by the gaming engine 204) to update the game state accordingly. It is to be noted by a person having ordinary skill in the art of person skilled in the art that the example of 3x5 grid of sensors shall not be construed as limiting the scope of the present disclosure.

The interactive table is equipped with a grid of sensors arranged in a 3x5 matrix format. It is to be noted by a person having ordinary skill in the art of person skilled in the art that the example of 3x5 matrix format shall not be construed as limiting the scope of the present disclosure. The sensors are capable of detecting taps or touches from the user, sending the corresponding data to the software component (represented by the gaming engine 204).

At step 304 of the method 300, the one or more hardware processors 102 place an object (represented by the object module 212) at an initial position specific to a position of the one or more sensors.

The software component (represented by the gaming engine 204) is structured as an interactive game where an object is initially placed at the bottom of a screen of the gaming engine 204. A virtual hand is displayed on the screen of the gaming engine 204, controlled by capturing the user's hand movements through readings of the one or more sensors. The interactive game includes a timer (represented by the timer 214) set to a pre-defined time interval (e.g., say 10 seconds), which is used to assess the user's reflexes.

Sensor Grid:

- Let the grid be defined as G where $G(i,j)$ represents the sensor at row $i$ and column $j$ of the grid, with $1 \leq i \leq 3$ and $1 \leq j \leq 5$.
- Each sensor $G(i,j)$ can detect user interaction and send its status to the software component (represented by the gaming engine 204).

Initial Setup: The object is initially placed at position $P_0 = (x_0, y_0)$, in the bottom part of the screen of the gaming engine 204 in a way, that the system 100 can understand cognitive capability. For example, object of interest is placed in the right most part of the screen and sensor is placed in the left most part of the table. A timer $T_0 = 10$ seconds (e.g., pre-defined time interval) is set to measure the user's reflex time. Herein the object can be one or more obstacles or reward coins. The one or more obstacles are placed in the easy positions (initial positions). Easy positions are designed as the place where the user can easily attain. The one or more obstacles might be some kind of blockers in the game and the reward coins are the way to increase their points in the game.

At step 306 of the method 300, the one or more hardware processors 102 detect one or more inputs by the one or more sensors from the user. The one or more inputs are obtained from a device associated with the user. The device includes at least one of a camera and an Infrared Sensor (IR) sensor.

User Interaction: The user's hand movement is tracked using a camera or Infrared Sensor (IR) sensor and represented as a virtual hand/character on the screen. The user must move his/her hand to touch the object within the given time $T_n$.

At step 308 of the method 300, the one or more hardware processors 102 send data pertaining to the detected one or more inputs obtained from the device associated with the user to a gaming engine (represented by the gaming engine 204) connected to the designed hardware component. Herein the detected one or more inputs can be the one or more touches, or the one or more taps made by the user. In the present disclosure, the one or more touches, or the one or more taps are made by the hands of the user. However, the one or more touches, or the one or more taps can also be made by leg movements or body movements of the user.

At step 310 of the method 300, the one or more hardware processors 102 records a reflex time pertaining to the user, when the user provides one or more inputs to the one or more sensors.

At step 312 of the method 300, the one or more hardware processors 102 perform a comparison of the reflex time within a predefined time $T_n$. The predefined time is set at $T_n = 10$ seconds initially. Based on the comparison, the system 100 performs one of the following steps. The initial position of the object is changed incrementally. Further the timer is reset to a predefined time interval. Herein, the predefined time interval $T_0 = 10$ seconds. The timer is increased with a first delta value (small incremental value). The position of the object is moved closer to the initial position with a second delta value (small incremental value).

If the user successfully moves their hand and taps/touches the object within the given time, the object is relocated to a different position on the screen, and the timer is reset to 10 seconds. The new position is determined incrementally based on the user's previous performance. If the user fails to touch the object within the allotted time, the system 100 either increases the timer value or repositions the object closer to its previous location with a small incremental adjustment. This feedback mechanism ensures that the game adapts to the user's abilities, providing a personalized experience.

Success Condition: If the user touches the object within $T_n$ seconds, the object is placed at a new position $P_n$ + 1:

$$P_n + 1 = P_n + \Delta P$$

where $\Delta P = (\Delta x, \Delta y)$ is the incremental change in position. The timer is reset to $T_0$=10 seconds.

Failure Condition: If the user fails to touch the object within $T_n$ seconds, the timer is adjusted:

$$T_{n+1} = T_n + \Delta T_2$$

or the object is moved closer to the initial position with a small incremental value:

$$P_{n+1} = P_n + \epsilon \cdot \Delta P$$

where $\epsilon$ is a factor (e.g., $0 < \epsilon < 1$).

The adaptive feedback module 206 provides adaptive feedback based on the user performance by adjusting the predefined time $T_n$. Adaptive feedback is processed in the game as the placement of obstacles / reward coins. If the user is performing/playing well then, the reward coin might be placed a further / challenging positions. It will encourage the user to play these games. Also, an audio-based feedback is provided to the user, based on the performance. The obstacles in the games are the way to understand the user's level of cognitive decline. For instance, if the user is not able to collect one coin or cover one obstacle then it is a way to indicate the user's cognitive decline level. By providing these kinds of adjustments in the game will motivate them to play these games. This is called adaptive feedback because these changes made during the game are based on the user input. The cognitive decline of the user can be memory loss and trouble concentrating, completing tasks, understanding, remembering, following instructions, and solving problems. Further, the cognitive decline conditions of the user can include dementia, Alzheimer's disease, Parkinson's disease, Dementia and the like.

Adaptive Feedback: The time $T_n$ can be adjusted based on user performance:

$$T_{n+1} = T_n - \Delta T_1 \text{ (if successful)}$$

$$T_{n+1} = T_n + \Delta T_2 \text{ (if failed)}$$

where $\Delta T_1$ and $\Delta T_2$ are positive constants.

At step 314 of the method 300, the one or more hardware processors 102 calculate the following based on the reflex time of the user. An average response time over $N$ inputs is evaluated, wherein a response time $T_i$ of the user is recorded for each of the one or more inputs. A progression metric is calculated to evaluate improvement based on a number of successful inputs from a number of total inputs. One or more adjustments to the timer are calculated based on the progression metric.

Recording and Analysis: User response time $t_i$ for each attempt is recorded. Average response time over $N$ attempts:

$$\bar{t} = \frac{1}{N} \sum_{i=1}^{N} t_i$$

Progression metric to evaluate improvement:

$$S_n \frac{Number\ of\ Successful\ attempts}{Total\ attempts}$$

Adjustments to $\Delta T_1$ and $\Delta T_2$ based on progression is expressed as below:

$$\Delta T_1 = f(S_n)$$

$$\Delta T_2 = g(S_n)$$

At step 316 of the method 300, the cognitive function deriving module 208 executed via the one or more hardware processors 102 derives a cognitive function to assess a the severity cognitive decline in the user based on the calculated average response time, the progression metric and the one or more adjustments.

Cognitive Assessment: A function to assess the cognitive decline severity of the user is based on response rate and progression which is expressed as below:

$$D = h(\bar{t}, S_n)$$

The system 100 positions the objects of interest in a way that the cognitive decline condition of the user is tested. Further the system 100 records the user's response times, success rates, and the adjustments made to the game parameters. This data is analyzed to evaluate the user's cognitive function and progression, particularly in relation to their cognitive decline condition. The game adapts in real-time based on the user's performance, either increasing or decreasing the difficulty to match their capabilities.

By monitoring these interactions, the system 100 not only provides therapeutic benefits but also offers a method for assessing the severity of the cognitive decline in users. The proposed invention thus serves as both an engaging therapy tool and a cognitive assessment device.

In the present disclosure, the average response time and the progression metric are used to understand the level of the cognitive decline associated with the user. The level of the cognitive decline can be expressed in terms of low, medium and high. The one or more adjustments made to the timer are used to place the one or more obstacles or reward coins in the game to encourage the user and reduce the complexity of the game. In this way, the one or more adjustments made to the timer are used to assess the cognitive decline level. For instance, if the user is not able to catch one reward coin every time, then the system 100 will place the reward coin in a lower place or in an easy location on the screen of the gaming engine 204. Further, if the user is still facing troubles to collect the reward coin, then it indicates the user has some level of the cognitive decline.

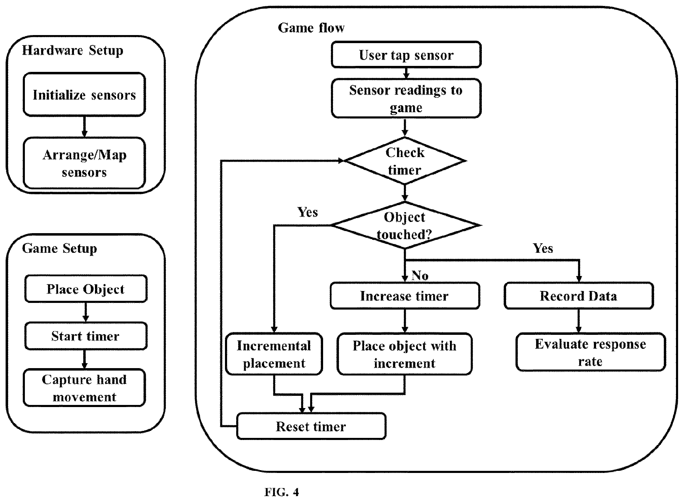

FIG. 4 shows a flow chart illustrating a hardware setup, a game flow, and a game set up in conjunction with the method to elder friendly device to improve hand eye co-ordination using an adaptive gaming technique, according to some embodiments of the present disclosure.

The written description describes the subject matter herein to enable any person skilled in the art to make and use the embodiments. The scope of the subject matter embodiments is defined by the claims and may include other modifications that occur to those skilled in the art. Such other modifications are intended to be within the scope of the claims if they have similar elements that do not differ from the literal language of the claims or if they include equivalent elements with insubstantial differences from the literal language of the claims.

Existing therapeutic methods lack interactivity and adaptability to individual needs. The embodiments of present disclosure address these issues by creating an adaptive game that not only provides engaging therapy but also monitors and assesses cognitive functions. The embodiment thus provides an interactive system designed to improve hand-eye coordination in individuals with the cognitive decline condition, consisting of an interactive table with a grid of sensors and a software component running an adaptive game. The game adapts based on the user's performance, adjusting the position of objects and the timer based on real-time sensor data. The system records user responses and progression metrics to assess cognitive functions, providing both therapeutic benefits and a method for cognitive assessment.

It is to be understood that the scope of the protection is extended to such a program and in addition to a computer-readable means having a message therein; such computer-readable storage means contain program-code means for implementation of one or more steps of the method, when the program runs on a server or mobile device or any suitable programmable device.

The hardware device can be any kind of device which can be programmed including e.g., any kind of computer like a server or a personal computer, or the like, or any combination thereof. The device may also include means which could be e.g., hardware means like e.g., an application-specific integrated circuit (ASIC), a field-programmable gate array (FPGA), or a combination of hardware and software means, e.g., an ASIC and an FPGA, or at least one microprocessor and at least one memory with software processing components located therein. Thus, the means can include both hardware means and software means. The method embodiments described herein could be implemented in hardware and software. The device may also include software means. Alternatively, the embodiments may be implemented on different hardware devices, e.g., using a plurality of CPUs.

The embodiments herein can comprise hardware and software elements. The embodiments that are implemented in software include but are not limited to, firmware, resident software, microcode, etc. The functions performed by various components described herein may be implemented in other components or combinations of other components. For the purposes of this description, a computer-usable or computer readable medium can be any apparatus that can comprise, store, communicate, propagate, or transport the program for use by or in connection with the instruction execution system, apparatus, or device.

The illustrated steps are set out to explain the exemplary embodiments shown, and it should be anticipated that ongoing technological development will change the manner in which particular functions are performed. These examples are presented herein for purposes of illustration, and not limitation. Further, the boundaries of the functional building blocks have been arbitrarily defined herein for the convenience of the description. Alternative boundaries can be defined so long as the specified functions and relationships thereof are appropriately performed. Alternatives (including equivalents, extensions, variations, deviations, etc., of those described herein) will be apparent to persons skilled in the relevant art(s) based on the teachings contained herein. Such alternatives fall within the scope of the disclosed embodiments. Also, the words "comprising," "having," "containing," and "including," and other similar forms are intended to be equivalent in meaning and be open ended in that an item or items following any one of these words is not meant to be an exhaustive listing of such item or items, or meant to be limited to only the listed item or items. It must also be noted that as used herein and in the appended claims, the singular forms "a," "an," and "the" include plural references unless the context clearly dictates otherwise.

Furthermore, one or more computer-readable storage media may be utilized in implementing embodiments consistent with the present disclosure. A computer-readable storage medium refers to any type of physical memory on which information or data readable by a processor may be stored. Thus, a computer-readable storage medium may store instructions for execution by one or more processors, including instructions for causing the processor(s) to perform steps or stages consistent with the embodiments described herein. The term "computer-readable medium" should be understood to include tangible items and exclude carrier waves and transient signals, i.e., be non-transitory. Examples include random access memory (RAM), read-only memory (ROM), volatile memory, nonvolatile memory, hard drives, CD ROMs, DVDs, flash drives, disks, and any other known physical storage media.

It is intended that the disclosure and examples be considered as exemplary only, with a true scope of disclosed embodiments being indicated by the following claims.

**Claims**

1.  A processor implemented method (300), comprising:

    retrieving from a database (302), via one or more hardware processors, a hardware component design as an interactive table comprising a grid of one or more sensors arranged in a predefined matrix format;
    placing (304), via the one or more hardware processors, an object at an initial position specific to a position of the one or more sensors;
    detecting (306), via the one or more hardware processors, by the one or more sensors, one or more inputs obtained from a device associated with a user;
    sending (308), via the one or more hardware processors, by the one or more sensors, data pertaining to the detected one or more inputs obtained from the device associated with the user to a gaming engine connected to the designed hardware component;
    recording (310), via the one or more hardware processors, a reflex time pertaining to the user, when the user provides the one or more inputs to the one or more sensors;
    performing (312), via the one or more hardware processors, a comparison of the reflex time within a predefined time $T_n$, and performing one of:

    (i) incrementally changing the initial position of the object;
    (ii) resetting a timer $T_0$ to a predefined time interval; or

    a) increasing the timer with a first delta value; and
    b) moving the position of the object closer to the initial position with a second delta value;

    calculating (314), via the one or more hardware processors, at least one of the following based on the reflex time of the user:

    (i) an average response time over N inputs, wherein a response time $t_i$ of the user is recorded for each of the one or more inputs;
    (ii) a progression metric to evaluate an improvement based on a number of successful inputs from a number of total inputs; and
    (iii) one or more adjustments to the timer based on the progression metric; and

    deriving (316), via the one or more hardware processors, a cognitive function to assess severity of cognitive decline in the user based on the calculated average response time, the progression metric and the one or more adjustments.

2.  The processor implemented method as claimed in claim 1, wherein the device includes at least one of a camera and an Infrared Sensor (IR) sensor.

3.  The processor implemented method as claimed in claim 1, wherein adaptive feedback is provided based on the reflex time performance of the user by adjusting the predefined time $T_n$.

4.  A system (100), comprising:

    a memory (104) storing instructions;
    one or more communication interfaces (112); and
    one or more hardware processors (102) coupled to the memory (104) via the one or more communication interfaces (112), wherein the one or more hardware processors (102) are configured by the instructions to:

    retrieve from a database, a hardware component design as an interactive table comprising a grid of one or more sensors arranged in a pre-defined matrix format;
    place an object at an initial position specific to a position of the one or more sensors;
    detect by the one or more sensors, one or more inputs obtained from a device associated with a user;
    send by the one or more sensors, data pertaining to the detected one or more inputs obtained from the device associated with the user to a gaming engine connected to the designed hardware component;
    record a reflex time pertaining to the user, when the user provides one or more inputs the one or more sensors;

perform a comparison of the reflex time within a predefined time $T_n$, and performing one of:

(i) incrementally changing the initial position of the object;
(ii) resetting a timer $T_0$ to a predefined time interval; or

a) increasing the timer with a first delta value; and
b) moving the position of the object closer to the initial position with a second delta value;

calculate at least one of the following based on the reflex time of the user:

(i) an average response time over $N$ inputs, wherein a response time $t_i$ of the user is recorded for each of the one or more inputs;
(ii) a progression metric to evaluate an improvement based on a number of successful inputs from a number of total inputs; and
(iii) one or more adjustments to the timer based on the progression metric; and

derive a cognitive function to assess the severity of cognitive decline of the user based on the calculated average response time, the progression metric and the one or more adjustments.

5. The system as claimed in claim 4, wherein the device includes at least one of a camera and an Infrared Sensor (IR) sensor.

6. The system as claimed in claim 4, wherein adaptive feedback is provided based on the reflex time of the user by adjusting the predefined time $T_n$.

7. One or more non-transitory machine-readable information storage mediums comprising one or more instructions which when executed by one or more hardware processors cause:

retrieving from a database, a hardware component design as an interactive table comprising a grid of one or more sensors arranged in a predefined matrix format;
placing an object at an initial position specific to a position of the one or more sensors;
detecting by the one or more sensors, one or more inputs obtained from a device associated with a user;
sending by the one or more sensors, data pertaining to the detected one or more inputs obtained from the device associated with the user to a gaming engine connected to the designed hardware component;
recording a reflex time pertaining to the user, when the user provides the one or more inputs to the one or more sensors;
performing a comparison of the reflex time within a predefined time $T_n$, and performing one of:

(i) incrementally changing the initial position of the object;
(ii) resetting a timer $T_0$ to a predefined time interval; or

a) increasing the timer with a first delta value; and
b) moving the position of the object closer to the initial position with a second delta value;

calculating at least one of the following based on the reflex time of the user:

(i) an average response time over $N$ inputs, wherein a response time $t_i$ of the user is recorded for each of the one or more inputs;
(ii) a progression metric to evaluate an improvement based on a number of successful inputs from a number of total inputs; and
(iii) one or more adjustments to the timer based on the progression metric; and

deriving a cognitive function to assess severity of cognitive decline in the user based on the calculated average response time, the progression metric and the one or more adjustments.

8. The one or more non-transitory machine-readable information storage mediums as claimed in claim 7, wherein the device includes at least one of a camera and an Infrared Sensor (IR) sensor.

9. The one or more non-transitory machine-readable information storage mediums as claimed claim 7, wherein adaptive feedback is provided based on the reflex time performance of the user by adjusting the predefined time $T_n$.

FIG. 1

| HARDWARE COMPONENT 202 | GAMING ENGINE 204 | ADAPTIVE FEEDBACK MODULE 206 | COGNITIVE FUNCTION DERIVING MODULE 208 |
|---|---|---|---|
| SENSORS MODULE 210 | OBJECT MODULE 212 | | |
| | TIMER 214 | | |

FIG. 2

retrieving from a database a hardware component design as an interactive table comprising a grid of one or more sensors arranged in a pre-defined matrix format — 302

placing an object at an initial position specific to a position of the one or more sensors — 304

detecting, by the one or more sensors, one or more inputs obtained from a device associated with a user — 306

sending by the one or more sensors, data pertaining to the detected one or more inputs obtained from the device associated with the user to a gaming engine connected to the designed hardware component — 308

recording a reflex time pertaining to the user, when the user provides one or more inputs the one or more sensors — 310

A

300

FIG. 3A

A

performing a comparison of the reflex time within a predefined time $T_n$, and performing one of:

(i) incrementally changing the initial position of the object;

(ii) restting a timer to $T_0$ a predefined time interval; or

(a) increasing the timer with a first delta value; and

(b) moving the position of the object closer to the initial position with a second delta value;

312

B

300

FIG. 3B

B

calculating at least one of the following based on the reflex time of the user:
(i) an average response time over $N$ inputs, wherein a response time $T_i$ of the user is recorded for each of the one or more inputs;
(ii) a progression metric to evaluate an improvement based on a number of successful inputs from a number of total inputs; and
(iii) one or more adjustments to the timer based on the progression metric

314

deriving a cognitive function to assess a health condition severity of the user based on the calculated average response time, the progression metric and the one or more adjustments

316

FIG. 3C

300

**Game flow**

**Hardware Setup**

- Initialize sensors
- Arrange/Map sensors

**Game Setup**

- Place Object
- Start timer
- Capture hand movement

User tap sensor

Sensor readings to game

Check timer

Object touched?

Yes

No

Yes

Increase timer

Record Data

Incremental placement

Place object with increment

Evaluate response rate

Reset timer

FIG. 4

Europäisches
Patentamt

European
Patent Office

Office européen
des brevets

# EUROPEAN SEARCH REPORT

**Application Number**

**EP 25 20 3242**

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2013/288777 A1 (SHORT GREGORY T [US] ET AL) 31 October 2013 (2013-10-31) <br> * paragraph [0042] * <br> * paragraph [0045] - paragraph [0047] * <br> * paragraph [0051] * <br> * paragraph [0056] * <br> * paragraph [0084] * <br> * paragraph [0113] * <br> * claim 1 * <br> ----- | 1-9 | INV. <br> A63F13/798 <br> A63F13/214 <br> A63F13/42 <br> A63F13/44 <br> A63F13/46 |
| A | US 2015/258432 A1 (STAFFORD JEFFREY ROGER [US] ET AL) 17 September 2015 (2015-09-17) <br> * paragraph [0003] * <br> * figures 1-5 * <br> ----- | 1,4,7 | |
| A | AU 2016 228 778 A1 (AKILI INTERACTIVE LABS INC [US]) 28 September 2017 (2017-09-28) <br> * paragraph [0003]; claim 1 * <br> ----- | 1-9 | |

**TECHNICAL FIELDS SEARCHED (IPC)**

A63F

The present search report has been drawn up for all claims

| Place of search | Date of completion of the search | Examiner |
|---|---|---|
| The Hague | 23 February 2026 | Mikulastik, Patrick |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 20 3242

This annex lists the patent family members relating to the patent documents cited in the above-mentioned European search report.
The members are as contained in the European Patent Office EDP file on
The European Patent Office is in no way liable for these particulars which are merely given for the purpose of information.

23-02-2026

| Patent document cited in search report | Publication date | Patent family member(s) | Publication date |
|---|---|---|---|
| US 2013288777 A1 | 31-10-2013 | US 2013288777 A1 | 31-10-2013 |
| | | US 2016117029 A1 | 28-04-2016 |
| US 2015258432 A1 | 17-09-2015 | CN 106233227 A | 14-12-2016 |
| | | EP 3116616 A1 | 18-01-2017 |
| | | JP 6307627 B2 | 04-04-2018 |
| | | JP 6538897 B2 | 03-07-2019 |
| | | JP 2017516186 A | 15-06-2017 |
| | | JP 2018129043 A | 16-08-2018 |
| | | US 2015258432 A1 | 17-09-2015 |
| | | WO 2015139002 A1 | 17-09-2015 |
| AU 2016228778 A1 | 28-09-2017 | AU 2016228778 A1 | 28-09-2017 |
| | | AU 2021202325 A1 | 13-05-2021 |
| | | CA 2979390 A1 | 15-09-2016 |
| | | EP 3267891 A1 | 17-01-2018 |
| | | JP 2018512202 A | 17-05-2018 |
| | | JP 2021058681 A | 15-04-2021 |
| | | JP 2022097488 A | 30-06-2022 |
| | | KR 20180041089 A | 23-04-2018 |
| | | US 2016262680 A1 | 15-09-2016 |
| | | US 2022117528 A1 | 21-04-2022 |
| | | US 2024008799 A1 | 11-01-2024 |
| | | WO 2016145372 A1 | 15-09-2016 |

EPO FORM P0459

**EP 4 736 972 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- IN 202421083425